# EUROPÄISCHE PATENTSCHRIFT

(11) **EP 3 541 321 B1**
(45) Veröffentlichungstag und Bekanntmachung des Hinweises auf die Patenterteilung: **04.01.2023**
(21) Anmeldenummer: 17811858.4
(22) Anmeldetag: 14.11.2017
(51) Int. Cl.: A61F 2/07, A61F 2/06

(54) **MULTILUMENIMPLANTAT**
MULTI-LUMEN IMPLANT
IMPLANT MULTI-LUMIÈRE

(30) Priorität: 18.11.2016 DE 102016122223
(43) Veröffentlichungstag der Anmeldung: 25.09.2019
(73) Patentinhaber: Stental GmbH, 79539 Lörrach (DE)
(72) Erfinder: TESSAREK, Jörg, 49808 Lingen (DE); OBRADOVIC, Milisav, 79539 Lörrach (DE)
(74) Vertreter: Schneiders & Behrendt Bochum
(86) Internationale Anmeldenummer: PCT/EP2017/079148
(87) Internationale Veröffentlichungsnummer: WO 2018/091442

(56) Entgegenhaltungen:
- WO-A1-2014/197743
- DE-A1-102012 100 754
- US-A1- 2003 065 385
- US-A1- 2003 176 911
- US-A1- 2013 138 202
- US-A1- 2014 074 218

## Beschreibung

Die Erfindung betrifft ein Multilumenimplantat zum Einsatz in menschliche und tierische Gefäßsysteme, umfassend ein im Wesentlichen schlauchförmiges Element, das sich in einen proximalen und einen distalen Abschnitt gliedert, und mindestens einen Stent zur Fixierung des proximalen Abschnitts in einem Zielgefäß.

Eine der häufigsten Pathologien des menschlichen Gefäßsystems bilden Aneurysmen. Bei Aneurysmen handelt es sich um Aussackungen der Gefäßwand. Die spezifischen Ursachen können sehr unterschiedlich sein, jedoch kann man generell sagen, dass der betroffene Teil der Gefäßwand zunächst geschwächt ist und sich durch den konstant auf ihm ruhenden Blutdruck immer mehr weitet.

Aneurysmen können prinzipiell in allen Teilen des Körpers und an allen Stellen im Gefäß entstehen. Es gibt jedoch im menschlichen Körper bestimmte Stellen, die allein aufgrund ihrer Gefäßanatomie für die Bildung von Aneurysmen prädestiniert sind. Hierzu zählen beispielsweise alle Gefäßgabelungen, sogenannte Bifurkationen.

Die Gefahr, die von einem Aneurysma für die Gesundheit des Patienten ausgeht, ist zweigeteilt. Zum einen kann das Aneurysma an sich zu Problemen führen, wenn es beispielsweise auf andere Gefäße oder Organe drückt. Hierdurch kann es zu Schmerzen und/oder einer Unterversorgung anderer Körperbereiche und Organe kommen. Ist beispielsweise das Gehirn betroffen, so sind mehr oder weniger schwerwiegende neurologische Ausfälle die Folge.

Zum anderen kann es zu einer Ruptur des Aneurysmas kommen, das heißt, Aneurysmen können auch reißen. Dies ist insbesondere dann für den Patienten gefährlich, wenn sich das Aneurysma im Gehirn oder an einem großen Gefäß befindet, das viel Blut führt. Im Gehirn führen geplatzte Aneurysmen zu Gehirnblutungen, die je nach Größe des Gefäßes und Stärke der Blutung leichte bis schwerste neurologische Schäden nach sich ziehen können. Im schlimmsten Fall führt eine solche Gehirnblutung zum Tod des Patienten.

Reißt ein Aneurysma an einem großen Gefäß im Körper des Patienten, kann es binnen kürzester Zeit zu einem tödlichen Blutverlust kommen, beispielsweise bei Rupturen von Baucharterienaneurysmen oder auch thorakalen Aneurysmen.

Noch vor wenigen Jahren mussten viele Aneurysmen offen chirurgisch behandelt werden. Inzwischen steht eine recht große Bandbreite an alternativen Behandlungsoptionen und Medizinprodukten für die minimalinvasive und intravaskuläre Behandlung von Aneurysmen bereit.

Eine der bevorzugten minimalinvasiven, intravaskulären Behandlungsoptionen eines Aneurysmas ist das Einsetzen von Stent-Grafts. Ein Stent-Graft besteht aus einem Stent-Gerüst und einem Überzug, was in der Kombination zu einem schlauartigen Implantat führt. Zur Behandlung eines Aneurysmas wird mit einem solchen Stent-Graft der Bereich des Aneurysmas überbrückt, indem der Stent-Graft die gesunden Bereiche des Gefäßes verbindet und das Blut entsprechend im Bereich des Aneurysmas durch den Stent-Graft fließt. Hierdurch wird zum einen eine Ruptur des Aneurysma verhindert, zum anderen wird der Druck, den das Aneurysma auf andere Strukturen ausgeübt hat, weitestgehend eliminiert, da kein Blut mehr durch das Aneurysma fließt.

Problematisch wird das Einsetzen eines solchen Stent-Grafts in Form eines einfachen Schlauches vor allem dann, wenn im Bereich des Aneurysmas Seitenäste vom betroffenen Gefäß abgehen. Genau wie das Aneurysma selbst werden dann auch die in dem Bereich liegenden Seitenäste durch die Implantation eines Stent-Grafts von der Blutversorgung abgeschnitten. Entsprechend werden die von den Seitenästen zu versorgenden Bereiche und/oder Organe von der Blutzufuhr abgeschnitten.

Um die Seitengefäße nicht von der Blutzufuhr abzuschneiden, ist es notwendig, diese über Ableitungen des Stent-Grafts zu versorgen. Im Stand der Technik ist eine Vielzahl von Techniken bekannt, die hierfür Lösungsmöglichkeiten anbieten. Etabliert haben sich heute vor allem maßangefertigte oder modulare Implantate, die auf die Bedürfnisse jedes einzelnen Patienten konfektioniert werden. Solche Maßanfertigungen und auch modulare Systeme sind teuer. Speziell Maßanfertigungen benötigen Zeit, da sie individuell meist händisch angefertigt werden müssen. Modulare Systeme werden hingegen häufig während der Intervention vom Operateur im Patienten selbst zusammengefügt.

Die EP 2 749 251 B1 (Cook Medical Technologies LLC) offenbart beispielweise ein erweiterbares, aus Grundkörper und Ringen bestehendes Stent-Graft-Transplantat, das in gewisser Weise modular an die jeweilige Gefäßanatomie angepasst werden kann. Die EP 2 081 515 B1 (Cook Medical Technologies LLC) offenbart spezielle Fensteranordnung in Stent-Grafts, um hierüber möglichst universell Seitenäste an das Implantat anbinden zu können. Und die WO 2014/197743 A1 (Aortic Innovations Surena LLC) schließlich beansprucht ein variables Stent-Graft-System, das es durch seinen modularen Aufbau erlaubt, die Seitenastabgänge des Implantats in vorgegebenen Längsbahnen an die Gefäßanatomie des Patienten anzupassen. Weitere Multilumenimplantate sind aus der DE 10 2012 100754 A1 und US 2003/065385 A1 bekannt.

Alle genannten Systeme haben Nachteile. Die Implantation bedarf in der Regel eines erfahrenen Operateurs, in vielen Fällen ist sogar eine systembezogene Schulung notwendig, um die modular aufgebauten Implantate während der Intervention korrekt im Körper des Patienten zu konstruieren.

Auch die Implantation von Systemen, die individualisiert angefertigt werden, stellt den Operateur vor große Herausforderungen. Die anatomisch genaue Anpassung des Implantats an den Hauptast und die Seitenastzugänge verlangt vom Operateur, das Implantat sehr präzise in die Anatomie des Patienten einzupassen. Was am starren Model einfach erscheint, ist in der Praxis ein höchst diffiziles Unterfangen, da die Gefäße eben nicht starr sind und ihre relative Lage zueinander verändern können.

Die Folgen des Einsatzes solcher komplexer, seien es nun komplett konfektionierte oder modulare Systeme, sind - neben einer sich erhöhenden Wahrscheinlichkeit von *device failures* - eine mitunter lange und somit für den Patienten und das Operationsteam belastende Interventionsdauer.

Hinzu kommt, dass die bekannten Systeme in aller Regel vollständig im Rahmen einer einzigen Intervention implantiert werden müssen. Eine stufenweise Implantation, ob geplant oder als Reaktion auf den Zustand des Patienten, ist in aller Regel nicht oder zumindest nicht gefahrlos möglich, da nur ein vollständig implantiertes System das benötigte Resultat erbringt. So kann der Operateur kaum auf einen sich beispielsweise verschlechternden Zustand des Patienten während der Intervention dadurch reagieren, dass Teilschritte auf spätere Termine verschoben werden. Ebenso ist es regelmäßig nicht möglich, die Intervention von Beginn an stufenweise über mehrere Tage zu planen.

Auch die Zugangsmöglichkeiten bekannter Systeme sind durch ihre Konstruktion häufig beschränkt. In der Regel weisen diese Systeme aufgrund ihrer Komplexität auch im zusammengefalteten Zustand recht große Durchmesser auf und können nur durch entsprechend große Schleusen eingeführt werden. Dies macht einen armseitigen Zugang in der Regel unmöglich. Ebenso ist ein retrograder Zugang beispielsweise zur Verbindung der Seitenäste konstruktionsbedingt bei bekannten Systemen meist unmöglich.

Nachteilig an den bekannten Systemen ist auch, dass diese durch eine Migration (Verschiebung der Prothese), eine Texturschädigung oder das Voranschreiten der Erkrankung im oberen und unteren Versiegelungssegment eine Undichtigkeit, eine sogenannte Endoleckage, entwickeln können. Die Sanierung kann extrem aufwändig sein und in aller Regel nur offen chirurgisch erfolgen.

Ein entscheidender Nachteil der bekannten Systeme ist ferner die notwendige Individualisierung. Die bekannten Systeme, auch wenn sie teilmodular ausgeführt sind, müssen dennoch in den meisten Fällen für den Einzelfall händisch angefertigt werden. Dies nimmt standardmäßig Tage bis Wochen und in Ausnahmefällen auch mehrere Monate in Anspruch. Eine Notfallversorgung mit solchen Implantaten ist dadurch regelmäßig nicht möglich, was zum vermeidbaren Tod eines Patienten innerhalb der Wartezeit auf ein entsprechendes Implantat führen kann.

Es ist daher eine Aufgabe der Erfindung, ein Multilumenimplantat zur Verfügung zu stellen, dass aufgrund seiner universellen Einsatz- und Anschlussmöglichkeiten - ohne prä-interventionelle Konfektionierung oder Individualisierung - für die Behandlung einer Vielzahl von Patienten und Krankheitsbildern geeignet ist. Ein solches System eignet sich auch für Notfälle und kann standardmäßig in Krankenhäusern bevorratet werden.

Eine Aufgabe der vorliegenden Erfindung ist auch das Bereitstellen eines Multilumenimplantats mit einem möglichst geringen Durchmesser im zusammengefalteten Zustand, das entsprechend über eine Schleuse mit geringem Durchmesser eingeführt werden kann, sodass neben femoralen insbesondere auch armseitige Zugänge standardmäßig möglich sind.

Es ist ferner Aufgabe der vorliegenden Erfindung, ein Multilumenimplantat zur Verfügung zu stellen, das kompatibel zu gängigem Zubehör ist. Insbesondere soll für den Anschluss des Implantats an das weiterführende Gefäßsystem der Einsatz möglichst viele der bekannten gecoverten Stents und Stent-Grafts möglich sein. Ebenso sollen auch stufenweise Interventionen ermöglicht werden.

Eine weitere Aufgabe der Erfindung besteht darin, ein möglichst einfach konstruiertes und möglichst einfach zu implantierendes System zur Verfügung zu stellen. Hierdurch können sowohl die Kosten und Fehlerraten des Systems als auch die Interventionsdauer und Interventionsrisiken minimiert werden.

Auch ist es Aufgabe der Erfindung, ein Implantat zur Verfügung zu stellen, das zur Sanierung bekannter Systeme bei Defekt geeignet ist, indem es eine vom ursprünglichen System komplett unabhängige Abdichtung und eigene Verankerung bietet, sodass bereits liegende Prothesenanteile, beispielsweise eines Endografts, im Körper verbleiben können

Das erfindungsgemäße Implantat verfolgt zur Lösung der gestellten Aufgaben einen Ansatz, der sich konzeptionell deutlich von dem bekannter Implantate unterscheidet. Bekannte Implantate versuchen die Anatomie der geschädigten Gefäßregion nachzubilden, den zu sanierenden Bereich gleichsam auszukleiden. Entsprechend müssen diese Implantate, wie eingangs dargestellt, individuell gefertigt und genauestens implantiert werden. Ein Implantat gemäß der vorliegenden Erfindung hingegen stellt prinzipiell eine Art Verteilereinsatz dar, dessen Lumen über gecoverte Stents individuell mit den zu versorgenden Gefäßen verbunden werden können.

Gelöst wird diese Aufgabe durch ein Multilumenimplantat mit den Merkmalen des Anspruchs 1. Vorteilhafte Ausgestaltungen sind jeweils Gegenstand der abhängigen Ansprüche.

Erfindungsgemäß besteht das Implantat aus einem im Wesentlichen schlauchförmigen Element und mindestens einem Stent zur Fixierung des schlauchförmigen Elements in einem Gefäß.

Das schlauchförmige Element gliedert sich in einen proximalen und einen distalen Abschnitt, wobei sich der distale Abschnitt in wenigstens zwei Lumina verzweigt. In der vorliegenden Anmeldung bezeichnet distal immer den Teil einer Struktur, der in Richtung des Blutstroms weist, entsprechend bezeichnet proximal den Teil, der gegen die Richtung des Blutstroms weist.

Das schlauchförmige Element kann man sich Fingerhandschuh-artig vorstellen.

Der proximale Abschnitt des schlauchförmigen Elements entspricht bei solch einem Vergleich weitestgehend dem den Handteller umgebenden Teil des Handschuhs und der distale Abschnitt des schlauchförmigen Elements im Wesentlichen den Fingern des Handschuhs, wobei die den Fingern entsprechenden distalen Aufzweigungen des Implantats offen enden oder an ihren Enden öffenbar sind.

In einer bevorzugten Ausführungsform verzweigt sich das proximale Lumen distal in fünf Lumen, einem Hauptlumen und vier Nebenlumen, es sind jedoch problemlos auch andere Aufteilungen denkbar und zu realisieren. Das erfindungsgemäße Implantat ist keinesfalls auf Ausführungsformen beschränkt, die den Relationen, Dimensionen oder sonstigen Eigenschaften eines Fingerhandschuhs ähneln.

Das schlauchförmige Element kann mittels mindestens eines Stents auf der Innen- oder Außenseite des proximalen Abschnitts des schlauchförmigen Elements stromauf vom Aneurysma im Zielgefäß fixiert werden. Das schlauchförmige Element kann durch weitere Maßnahmen an dem Stent befestigt sein, beispielsweise durch Klammern, Verschweißen, Verkleben, Vernähen oder durch sonstige bekannte Maßnahmen. Besonders bevorzugt ist eine klammerartige Verbindung, wie sie in der WO 2012/084202 A2 offenbart wird. Es sind auch Ausführungsformen denkbar, bei denen sich der proximale Abschnitt des schlauchförmigen Elements zwischen zwei Stents befindet und gegebenenfalls zusätzlich, wie zuvor ausgeführt, an einem oder beiden Stents befestigt wird.

Denkbar sind in diesem Zusammenhang Ausführungsformen, bei denen sich der Stent nicht ausschließlich im proximalen Abschnitt des schlauchförmigen Elements befindet, sondern bis in den distalen Bereich oder sogar über das Ende des distalen Bereichs hinaus ragt. Hierbei kann beispielsweise auch ein mehr oder weniger langer erster Teil des proximalen Abschnitts des schlauchförmigen Elements nicht von dem Stent bedeckt sein und entsprechend kann dieser erste Abschnitt dann um das proximale Ende des Stents nach innen oder außen umgeschlagen werden.

Bevorzugt ist jedoch eine Ausführungsform, bei der der proximale Abschnitt des schlauchförmigen Elements zumindest teilweise auf dessen distalen Abschnitt umgeschlagen wird. In einer besonders bevorzugten Ausführungsform befindet sich der Stent zur Fixierung des schlauchförmigen Elements dann in dem - zwischen den ursprünglichen Außenseiten des proximalen und distalen Abschnitts des schlauchförmigen Elements - entstandenen Umschlag.

Weiterhin bevorzugt ist eine Ausführungsform im Sinne der vorangegangenen Beschreibung, bei der der proximale Abschnitt des schlauchförmigen Elements auf den distalen Abschnitt umgeschlagen wird und die Länge des umgeschlagenen proximalen Abschnitts die Länge des sich in dem entstandenen Umschlag befindlichen Stents übertrifft. So kann der distal über den Stent hinausragende Teil des proximalen Abschnitts über das distale Ende des Stents nach innen geschlagen werden kann, sodass der Stent anschließend an seinen beiden Enden vom proximalen Abschnitt des schlauchförmigen Elements ummantelt ist.

Der distale Abschnitt des schlauchförmigen Elements verzweigt sich in mindestens zwei Lumina, wobei die Zahl der Lumina beliebig ist und sich die Anzahl der Verzweigungen beispielsweise nach der Anzahl der zu versorgenden Gefäße richten kann. Bevorzugt sind jedoch Ausführungsformen mit fünf Verzweigungen.

Insbesondere bevorzugt sind Ausführungsformen mit einem Hauptlumen mit einem größeren und vier Nebenlumina mit einem kleineren Durchmesser. Die Durchmesser sind jedoch grundsätzlich beliebig und können entsprechend des Einsatzzwecks des Implantats gewählt werden. So sind beim Einsatz des Implantats im thorakalen und abdominalen Bereich Durchmesser des proximalen Abschnitts zwischen 5 und 45 mm, bevorzugt zwischen 20 und 42 mm möglich, für das Hauptlumen zwischen 3 und 30 mm, bevorzugt zwischen 12 und 25 mm möglich, und für die Nebenlumina zwischen 2 und 12 mm, bevorzugt zwischen 4 und 10 mm möglich. Beim Einsatz des Implantats im zerebralen Bereich sind Durchmesser des proximalen Abschnitts zwischen 2 und 15 mm, bevorzugt zwischen 2 und 8 mm möglich, für das Hauptlumen zwischen 1 und 5 mm, bevorzugt zwischen 2 und 4 mm möglich, und für die Nebenlumina zwischen 1 und 4 mm, bevorzugt zwischen 2 und 3 mm möglich. Beim Einsatz des Implantats im koronaren Bereich sind Durchmesser des proximalen Abschnitts zwischen 2 und 8 mm, bevorzugt zwischen 4 und 6 mm möglich, für das Hauptlumen zwischen 2 und 6 mm, bevorzugt zwischen 3 und 5 mm möglich, und für die Nebenlumina zwischen 2 und 5 mm, bevorzugt zwischen 2 und 3 mm möglich.

Es sind neben den genannten auch Ausführungsformen denkbar, die über insgesamt mehr oder weniger als fünf Lumina verfügen, weiterhin sind auch Ausführungsformen denkbar, die über mehr als ein Hauptlumen und über mehr oder weniger als vier Nebenlumina verfügen.

Denkt man sich einen Querschnitt durch einen Bereich des Implantats nach der Aufzweigung, so ist sind Ausführungsformen mit einer exzentrischen Verteilung der Lumina bevorzugt, bei der sich die Aufzweigung mit dem größten Lumen auf einer und eine oder mehrere Aufzweigungen mit kleineren Lumen auf der anderen Seite im Querschnitt befinden. Die Verteilung der Aufzweigungen im distalen Abschnitt des schlauchförmigen Elements ist jedoch nicht festgelegt und es sind auch Ausführungsformen denkbar, bei denen sich beispielsweise eine Aufzweigung im zentralen Bereich befindet und sich weitere Aufzweigungen gleich- oder ungleichverteilt um das zentrale Lumen anordnen.

In einer bevorzugten Ausführungsform sin die einzelnen Aufzweigungen gleich lang. Es sind jedoch auch Ausführungsformen denkbar, bei denen die Aufzweigungen unterschiedlich lang sind. Insgesamt ist es bevorzugt, wenn die Aufzweigungen distal ungefähr mit dem Stent zur Fixierung des Implantats enden. Es ist jedoch denkbar, dass die Aufzweigungen auch distal von dem Stent überragt werden oder distal aus dem Stentgerüst herausragen.

Die Aufzweigungen des Implantats werden über gecoverte Stents, sogenannte Stentgrafts, mit den distal zum Implantat liegenden Gefäßen verbunden, wodurch der Bereich des Aneurysmas überbrückt, der Bluteinstrom in das Aneurysma verhindert und das Aneurysma entlastet wird. Zugleich werden die distalen Gefäße gezielt über die Stents mit Blut versorgt.

Zur besseren Fixierung der Stents, die die distalen Aufzweigungen des schlauchförmigen Elements mit den jeweils zu versorgenden Gefäßen verbinden, können sich die distalen Aufzweigungen in einer bevorzugten Ausführungsform zu ihrem Ende hin insgesamt oder in einem bestimmten Abschnitt konisch verjüngen.

Weitere Maßnahmen zur Sicherung der Stents in den distalen Aufzweigungen sind in Form von ringförmigen oder sonstigen Verstärkungen oder Versteifungen insbesondere der distalen Bereiche denkbar. Hierdurch wird zum einen der feste Sitz des Stentgrafts gewährleistet, ferner wird durch diese Verstärkung auch die Gefahr eines Reißens der distalen Aufzweigungen bei der Implantation der Stentgrafts verringert.

Die Aufzweigungen im distalen Abschnitt verlaufen bevorzugt frei voneinander, es sind jedoch auch Ausführungsformen denkbar, bei denen alle oder einige von ihnen untereinander, gegebenenfalls auch in Gruppen, verbunden sind. Die Aufzweigungen können auch - zusätzlich oder alternativ - an dem zur Fixierung des Implantats dienenden Stent oder an dem umgeklappten proximalen Abschnitt des schlauchförmigen Elements befestigt sein.

Als Materialien für das schlauchförmige Element kommen alle im Stand der Technik bekannten physiologisch verträglichen Materialien in Frage, vor allem ePTFE, die auch durch Elektrospinnen verarbeitet sein können.

Für den oder die Stents kommen alle im Stand der Technik bekannten Materialien in Frage, die dauerhaft eine Fixierung des schlauchförmigen Elements gewährleisten und nicht resorbierbar sind. Geeignet sind hierfür sowohl Legierungen, die eine Ballonexpansion ermöglichen, als auch Formgedächtnismaterialien, wie beispielsweise Nickel-Titan-Legierungen.

Typischerweise wird das erfindungsgemäße Implantat am proximalen Ende des Aneurysmas in den dort noch intakten Gefäßabschnitt eingesetzt. Das Implantat erfüllt hier die Funktion einer Verteilerscheibe, die das Blut nur durch die Lumina der distalen Aufzweigungen fließen lässt. Die distalen Aufzweigungen des schlauchförmigen Elements werden über gecoverte Stents mit den distal des Implantats liegenden Gefäßen verbunden. So wird der Bereich des Aneurysmas überbrückt.

Durch diese Technik ist es möglich, die Intervention auch etappenweise durchzuführen, da der Einsatz des Implantats zunächst die Blutzufuhr in die distal des Implantats gelegenen Gefäße nicht unterbindet. So kann beispielsweise in einer ersten Intervention das Implantat an sich eingesetzt werden und in einer oder mehreren weiteren Interventionen werden die Gefäße angeschlossen.

Auch ist ein bei bekannten Implantaten nur schwierig oder gar nicht realisierbarer retrograder Zugang möglich, um die Gefäße und Aufzweigungen des Implantats miteinander zu verbinden.

Das erfindungsgemäße Multilumenimplantat ermöglicht aufgrund der universellen Adaptierbarkeit auch die Reparatur bereits liegender Systeme, die durch eine Migration (Verschiebung der Prothese), eine Texturschädigung oder das Voranschreiten der Erkrankung im oberen und unteren Versiegelungssegment eine Undichtigkeit, eine sogenannte Endoleckage, entwickelt haben. Die bereits liegenden Prothesenanteile können im Körper verbleiben, da das Multilumenimplantat eine komplett unabhängige Abdichtung und eigene Verankerung bietet.

Die Vorteile, die ein erfindungsgemäßes Implantat bietet, sind jedoch noch weitaus vielfältiger. Sie betreffen auch die Herstellungskosten und somit die Kosten für das Gesundheitssystem, die Patientensicherheit, die Planbarkeit, die Verfügbarkeit, die Produktsicherheit und auch die Sicherheit der Intervention.

Das Implantat ist aufgrund seiner universellen Einsetzbarkeit durch die Standardisierung auf bevorzugt fünf distale Lumina in größeren Stückzahlen zu entsprechend niedrigeren Stückkosten produzierbar und somit gegenüber individualisierten Implantaten deutlich günstiger.

Da das Implantat durch seine flexiblen Anschlussmöglichkeiten universell einsetzbar ist, kann es in der Klinik bevorratet und so auch bei akuten Notfällen eingesetzt werden. Eine individuelle Anfertigung mit teils langen Wartezeiten ist nicht notwendig.

Das Implantat ist einfach zu implantieren, es muss weder zuvor konfektioniert noch im Gefäßsystem des Patienten montiert werden. Prinzipiell erfolgt die Implantation analog zum Einsetzen eines gewöhnlichen Stents. Auch das Anschließen der Gefäße an die Aufzweigungen erfordert keine besonderen Kenntnisse, es entspricht dem routinemäßigen Einsetzen von Stentgrafts.

Die Einfachheit des Implantats macht es gegenüber bekannten Implantaten auch weniger anfällig für *device failures,* die im Rahmen der Intervention oder der vorherigen Anfertigung und Konfektionierung begründet sein können.

So ist nicht zuletzt auch von einer erhöhten Sicherheit der eigentlichen Intervention auszugehen, da sich aufgrund der Einfachheit der Intervention, es entfällt der komplizierte Anschluss der Gefäße und die Montage des Implantats im Patienten, die Interventionszeit und somit automatisch auch das Risiko von operationsbegleitenden Komplikationen senken werden.

Die Erfindung sowie das technische Umfeld werden nachfolgend anhand der Figuren näher erläutert. Es ist darauf hinzuweisen, dass die Figuren eine besonders bevorzugte Ausführungsvariante der Erfindung zeigen. Die Erfindung ist jedoch nicht auf die gezeigte Ausführungsvariante beschränkt.

Es zeigen:
- Fig. 1: eine schematische Darstellung des schlauchförmigen Elements.
- Fig. 2: eine schematische Darstellung einer ersten Ausführungsform des erfindungsgemäßen Implantats.
- Fig. 3a-b: eine schematische Darstellung des Umklappens des proximalen auf den distalen Abschnitt zum besseren Verständnis einer zweiten Ausführungsform des erfindungsgemäßen Implantats.
- Fig. 4: eine schematische Darstellung des weiteren Umklappens des proximalen auf den distalen Abschnitt und der Position des Stents zum besseren Verständnis einer zweiten Ausführungsform des erfindungsgemäßen Implantats.
- Fig. 5: eine schematische Darstellung einer zweiten Ausführungsform des erfindungsgemäßen Implantats.
- Fig. 6a-b: einen a) Quer- und einen b) Längsschnitt durch eine zweite Ausführungsform des erfindungsgemäßen Implantats.
- Fig. 7a-d: eine schematische Darstellung möglicher Verteilungsoptionen der distalen Lumina.
- Fig. 8a-d: mögliche Ausgestaltungen der distalen Enden der Aufzweigungen zur besseren Fixierung der weiterleitenden Stents.

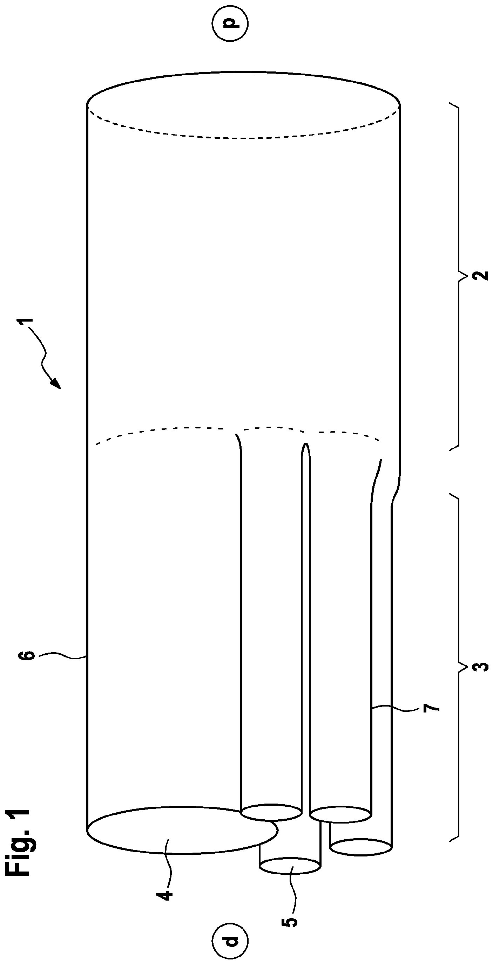

Figur 1 zeigt schematisch das schlauchförmige Element 1 eines erfindungsgemäßen Implantats, das sich in einen proximalen Abschnitt 2 und einen distalen Abschnitt 3 gliedert. Das proximal p einlumige schlauchförmige Element 1 verzweigt sich distal d in mindestens zwei Lumina 4, 5, wobei es in einer bevorzugten Ausführungsform ein Hauptlumen 4 und vier Nebenlumina 5 gibt, oder entsprechend eine große Aufzweigung 6 und vier kleinere Aufzweigungen 7.

Die relativen Größenverhältnisse dieser schematischen Darstellung beruhen rein auf einer guten Erkennbarkeit der einzelnen Teile. So können sowohl das Verhältnis von proximalem 2 zu distalem Abschnitt 3 als auch die Verhältnisse der einzelnen distalen Lumina 4 und 5 oder der Längen der Aufzweigungen 6 und 7 andere sein, als hier dargestellt.

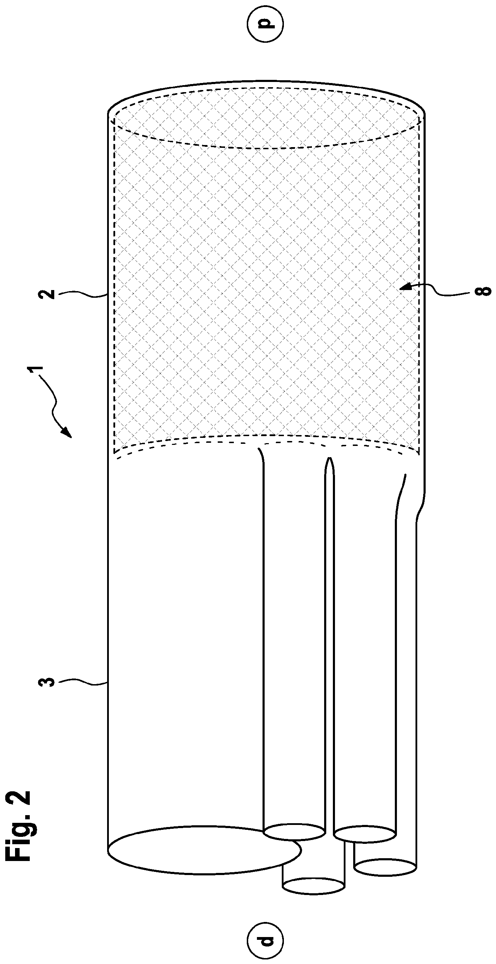

Figur 2 zeigt das schlauchförmige Element 1 aus Figur 1, wobei sich nun ein Stent 8 zur Festlegung beziehungsweise Fixierung des schlauchförmigen Elements 1 in einem Gefäß im Inneren des proximalen Abschnitts 2 befindet. Der Stent 8 reicht bei dieser Ausführungsform bis an den distalen Abschnitt 3 heran. Das proximale Ende des schlauchförmigen Elements 1 kann den Stent 8 gegebenenfalls überragen und über das proximale Stentende nach innen in das Lumen des proximalen Abschnitts 2 umgeschlagen werden.

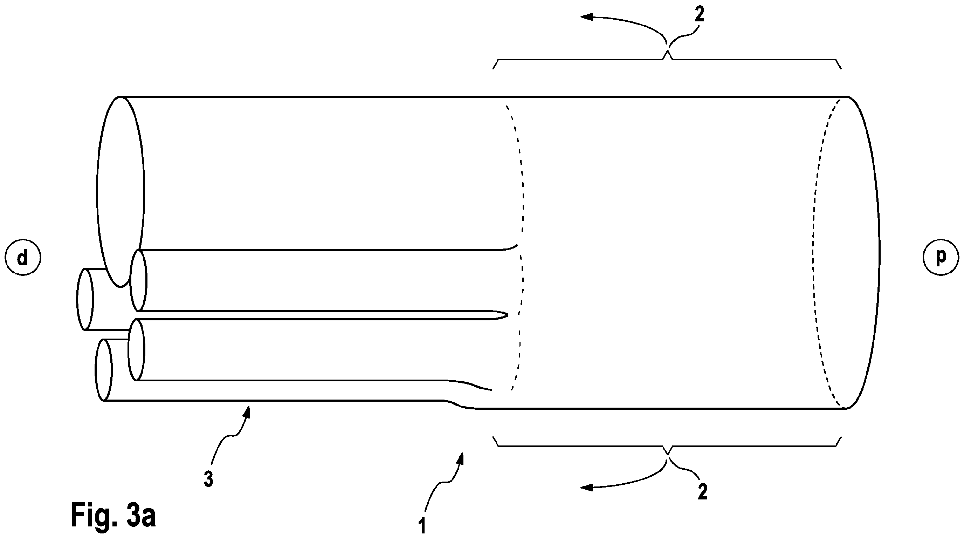

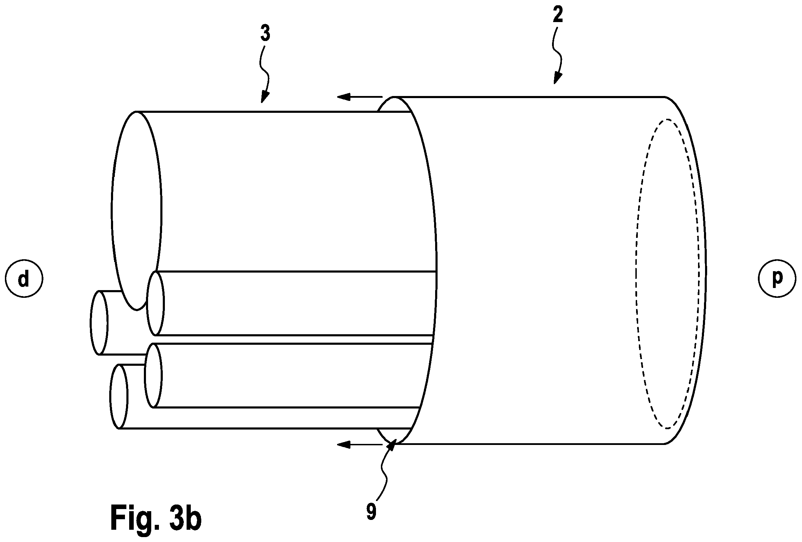

Figuren 3a und 3b verdeutlichen die Anordnung des proximalen Abschnitts 2 des schlauchförmigen Elements 1 in einer ersten bevorzugten Ausführungsform. Hier wird der proximale Abschnitt 2 auf den distalen Abschnitt 3 umgeschlagen, sodass der distale Abschnitt 3 schließlich zumindest teilweise vom proximalen Abschnitt 2 ummantelt wird.

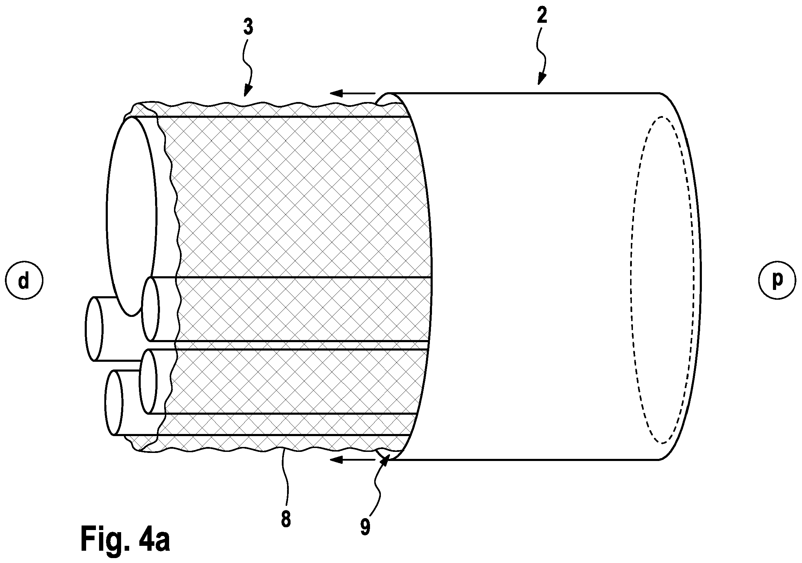

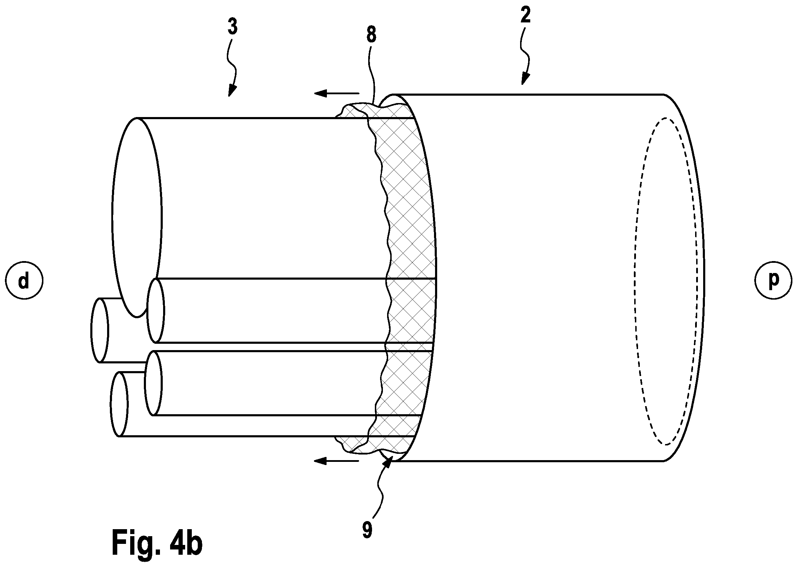

Figuren 4a und 4b zeigen zwei Varianten einer ersten bevorzugten Ausführungsform, die auf dem Umschlagen des proximalen Abschnitts 2 wie in den Figuren 3a und 3b dargestellt beruhen. Dabei unterscheiden sich die dargestellten Varianten 4a und 4b in der Länge des Stents 8, der sich jeweils zum einen im durch das Umschlagen gebildeten Umschlag 9 befindet, jedoch unterschiedlich weit nach distal d aus diesem herausragt und entsprechend den distalen Abschnitt 3 mehr 4a oder weniger 4b überragt.

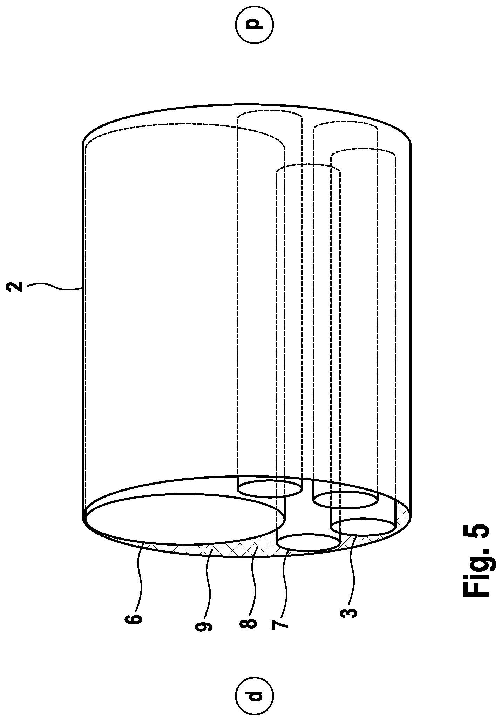

Figur 5 zeigt eine bevorzugte Ausführungsform, die auf dem Umschlagen des proximalen Abschnitts 2 wie in den Figuren 3a und 3b dargestellt beruht. Der proximale Abschnitt 2 wird nach distal d bis an das Ende der Aufzweigungen 6, 7 umgeschlagen. In dem so entstandenen Umschlag 9 befindet sich ein Stent 8. Die Längen des Stents 8, des proximalen 2 und des distalen 3 Abschnitts sind in dieser Darstellung in etwa gleich, sodass der Stent 8 komplett innerhalb des Umschlags 9 liegt.

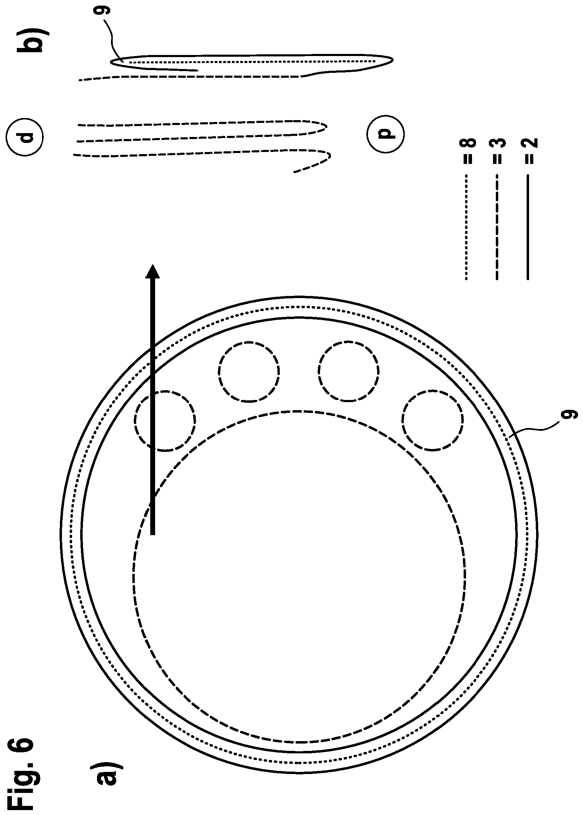

Figur 6 zeigt einen Querschnitt 6a und einen Längsschnitt 6b durch die in Figur 5 dargestellte bevorzugte Ausführungsform des Implantats. Die gepunktete Linie kennzeichnet den Stent 8, die gestrichelte Linie den distalen Abschnitt 3 und die durchgehende Linie den proximalen Abschnitt 2 des schlauchförmigen Elements 1. Wie in beiden Figuren zu erkennen ist, wird der proximale Abschnitt 2 nach distal d auf den distalen Abschnitt 3 umgeschlagen und dann distal d um den sich in dem Umschlag 9 befindlichen Stent 8 innen nach proximal p umgeschlagen, sodass der Stent 8 zumindest im distalen Bereich auf beiden Seiten vom proximalen Abschnitt 2 bedeckt ist.

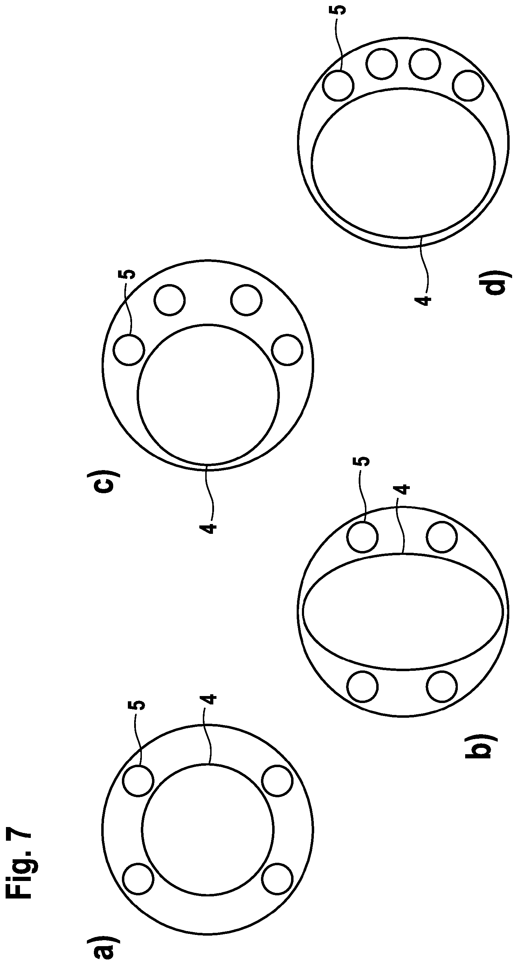

Figur 7 zeigt rein schematisch als Querschnitte durch das Implantat verschiedene Aufteilungen und Anordnungen der distalen Lumina 4, 5. Das Hauptlumen 4 kann zentral a, b oder exzentrisch c, d angeordnet sein. Die Nebenlumina 5 können entsprechend um das Hauptlumen 4 herum a, b oder auf einer Seite c, d angeordnet sein. Das Hauptlumen 4 kann im Querschnitt rund a, c oder oval b, d sein. Ebenso sind Ausführungsformen denkbar, bei denen auch die Nebenlumina 5 oval sind. Eine bevorzugte Ausführungsform ist in Figur 7c dargestellt.

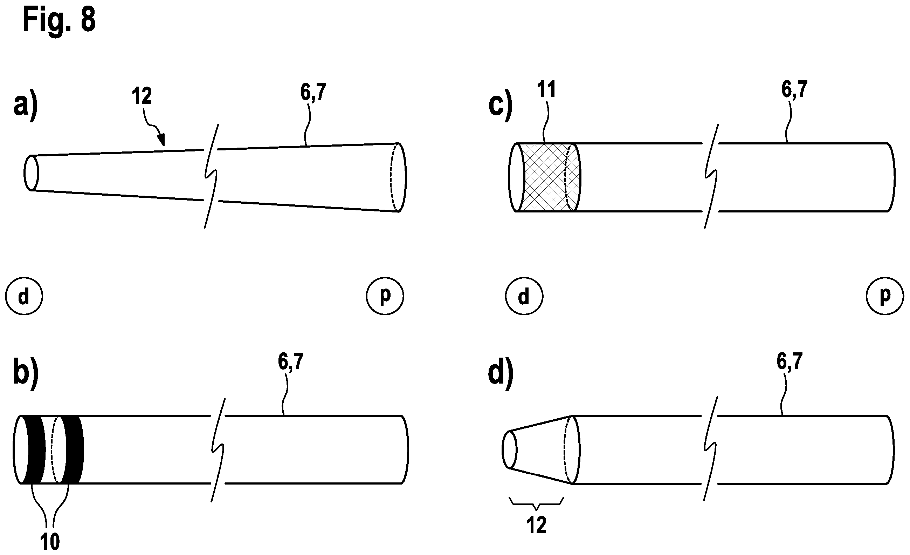

Figur 8 zeigt verschiedene Ausgestaltungsmöglichkeiten der distalen Enden d der distalen Aufzweigungen 6, 7 zur besseren Fixierung der Stents, die die distalen Aufzweigungen 6, 7 mit den jeweils zu versorgenden Gefäßen verbinden. Hierzu können sich die distalen Aufzweigungen in einer bevorzugten Ausführungsform zu ihrem Ende hin insgesamt a oder an der distalen Spitze d, 12 konisch verjüngen, an der Spitze über ringförmige 10 Verfestigungen oder eine distal insgesamt verstärkte Struktur c, 11 verfügen.

### Bezugszeichenliste

- 1: Schlauchförmiges Element
- 2: Proximaler Abschnitt
- 3: Distaler Abschnitt
- 4: Hauptlumen
- 5: Nebenlumen
- 6: Hauptaufzweigung
- 7: Nebenaufzweigung
- 8: Stent
- 9: Umschlag
- 10: Ringförmige Verfestigung
- 11: Ringförmige Verstärkung
- 12: Verjüngung
- p: proximal
- d: distal

## Patentansprüche

1. Multilumenimplantat zum Einsatz in menschliche und tierische Gefäßsysteme/Körper, mit einem im Wesentlichen schlauchförmigen Element (1), das sich in einen proximalen (2) und einen distalen (3) Abschnitt gliedert, und mindestens einem Stent (8) zur Fixierung des proximalen Abschnitts (2) in einem Zielgefäß, wobei sich das schlauchförmige Element (1) im distalen Abschnitt (3) in wenigstens zwei Aufzweigungen (6, 7) mit entsprechenden Lumina (4, 5) verzweigt und wobei der proximale Abschnitt (2) des schlauchförmigen Elements (1) auf den distalen Abschnitt (3) umgeschlagen wird, so dass der Stent (8) zwischen dem proximalen Abschnitt (2) des schlauchförmigen Elements (1) und dem distalen Abschnitt (3) angeordnet ist, **dadurch gekennzeichnet, dass** die Länge des umgeschlagenen proximalen Abschnitts (2) des schlauchförmigen Elements (1) mindestens der Länge des Stents (8) entspricht und wobei die Aufzweigungen (6, 7) zumindest teilweise innerhalb des Stents (8) angeordnet sind.

2. Multilumenimplantat nach Anspruch 1, **dadurch gekennzeichnet, dass** sich das schlauchförmige Element (1) im distalen Abschnitt (3) in eine große Aufzweigung (6) mit einem Hauptlumen (4) und mehrere kleinere Aufzweigungen (7) mit Nebenlumina (5) verzweigt.

3. Multilumenimplantat nach Anspruch 2, **dadurch gekennzeichnet, dass** die Nebenlumina (5) ein kleineres Kaliber haben als das Hauptlumen (4).

4. Multilumenimplantat nach einem der Ansprüche 1 bis 3, **dadurch gekennzeichnet, dass** der Stent (8) auf der Innenseite des proximalen Abschnitts (2) des schlauchförmigen Elements (1) angeordnet ist.

5. Multilumenimplantat nach einem der Ansprüche 1 bis 3, **dadurch gekennzeichnet, dass** der Stent (8) auf der Außenseite des proximalen Abschnitts (2) des schlauchförmigen Elements (1) angeordnet ist, wobei der erste Teil des proximalen Abschnitts des schlauchförmigen Elements (1) zumindest teilweise um den proximalen Teil des Stents (8) außen nach distal herumgeführt ist.

6. Multilumenimplantat nach einem der vorherigen Ansprüche, **dadurch gekennzeichnet, dass** der proximale Abschnitt (2) des schlauchförmigen Elements (1) länger ist als der Stent (8) und dass der distal (d) über den Stent (8) ragende Anteil des proximalen Abschnitts (2) um den Stent (8) nach innen geschlagen wird.

7. Multilumenimplantat nach einem der vorherigen Ansprüche, **dadurch gekennzeichnet, dass** sich Aufzweigungen (6, 7) bzw. die Lumina (4, 5) des distalen Abschnitts (3) des schlauchförmigen Elements (1) nach distal (d) verjüngen (12).

8. Multilumenimplantat nach einem der vorherigen Ansprüche, **dadurch gekennzeichnet, dass** die Aufzweigungen (6, 7) distal über insbesondere ringförmige Verstärkungen (10, 11) verfügen.

9. Multilumenimplantat nach einem der vorherigen Ansprüche, **dadurch gekennzeichnet, dass** das schlauchförmige Element (1) am Stent (8) festgelegt ist durch Klemmen, Verkleben, Vernähen oder Verschweißen.

10. Multilumenimplantat nach einem der vorherigen Ansprüche, **dadurch gekennzeichnet, dass** das schlauförmige Element (1) aus ePTFE besteht.

11. Multilumenimplantat nach Anspruch 10, **dadurch gekennzeichnet, dass** das schlauförmige Element (1) aus ePTFE durch Elektrospinnen erhältlich ist.

12. Multilumenimplantat nach einem der vorherigen Ansprüche, **dadurch gekennzeichnet, dass** es sich bei dem Stent (8) um einen selbstexpandierenden Stent handelt.

13. Multilumenimplantat nach einem der Ansprüche 1 bis 11, **dadurch gekennzeichnet, dass** es sich bei dem Stent (8) um einen ballonexpandierenden Stent handelt.

14. Kit, umfassend ein Implantat gemäß den vorherigen Ansprüchen und einem Ballonkatheter und/oder Katheter zu dessen Implantation.

15. Kit gemäß Anspruch 14, weiterhin umfassend eine entsprechenden Anzahl von Stentgrafts, zum Anschluss der distalen Aufzweigungen (6, 7) des Implantats mit den distalen Gefäßen.

## Claims

1. Multilumen implant for the application in human and animal vascular systems/bodies, comprising a substantially tubular element (1) that divides into a proximal (2) and a distal section (3), and at least one stent (8) for the fixation of the proximal section (2) in a target vessel, wherein the tubular element (1) is designed to branch in the distal section (3) into at least two branches (6, 7) with respective lumens (4, 5) and wherein the proximal section (2) of the tubular element (1) is folded over upon the distal section (3) so that the stent (8) is arranged between the proximal section (2) of the tubular element (1) and the distal section (3) **characterized in that** the length of the folded proximal section (2) of the tubular element (1) corresponds to at least the length of the stent (8) and wherein the branches (6, 7) are at least partially arranged inside the stent (8).

2. Multilumen implant according to claim 1, **characterized in that** the tubular element (1) branches in the distal section (3) into a main branch (6) with a main lumen (4) and a plurality of secondary branches (7) with secondray lumens (5).

3. Multilumen implant according to claim 2, **characterized in that** the secondary lumens (5) have a smaller caliber than the main lumen (4).

4. Multilumen implant according to any one of claims 1 to 3, **characterized in that** the stent (8) is arranged on the inside of the proximal section (2) of the tubular element (1).

5. Multilumen implant according to any one of claims 1 to 3, **characterized in that** the stent (8) is arranged on the outside of the proximal section (2) of the tubular element (1), wherein the first part of the proximal section of the tubular element (1) being outwardly guided distally at least partially around the proximal part of the stent (8).

6. Multilumen implant according to any one of the preceding claims, **characterized in that** the proximal section (2) of the tubular element (1) is longer than the stent (8) and **in that** the portion of the proximal section (2) projecting distally (d) beyond the stent (8) is folded inwardly over the stent (8).

7. Multilumen implant according to any one of the preceding claims, **characterized in that** the branches (6, 7) and respectively the lumens (4, 5) of the distal section (3) of the tubular element (1) taper in distal direction (d) (12).

8. Multilumen implant according to any one of the preceding claims, **characterized in that** the branches (6, 7) have distally in particular annular reinforcements (10, 11).

9. Multilumen implant according to any one of the preceding claims, **characterized in that** the tubular element (1) is secured to the stent (8) by means of clamping, gluing, sewing or welding.

10. Multilumen implant according to any one of the preceding claims, **characterized in that** the tubular element (1) consists of ePTFE.

11. Multilumen implant according to claim 10, **characterized in that** the tubular element (1) made of ePTFE is produced by electrospinning.

12. Multilumen implant according to any one of the preceding claims, **characterized in that** the stent (8) is a self-expanding stent.

13. Multilumen implant according to any one of the claims 1 to 11, **characterized in that** the stent (8) is a balloon-expanding stent.

14. Kit comprising an implant according to the preceding claims and a balloon catheter and/or a catheter for its implantation.

15. Kit according to claim 14, moreover comprising a respective number of stent grafts for the purpose of connecting the distal branches (6, 7) of the implant to the distal vessels.

## Revendications

1. Implant multi-lumière destiné à être employé dans des systèmes vasculaires/des corps chez l'homme et l'animal, avec un élément sensiblement tubulaire (1), qui se structure en un segment proximal (2) et en un segment distal (3), et avec au moins une endoprothèse vasculaire (8) destinée à bloquer le segment proximal (2) dans un vaisseau cible, dans lequel l'élément tubulaire (1) dans le segment distal (3) se ramifie en au moins deux embranchements (6, 7) avec des lumières (4, 5) correspondantes et dans lequel le segment proximal (2) de l'élément tubulaire (1) est retourné sur le segment distal (3) de sorte que l'endoprothèse vasculaire (8) est disposée entre le segment proximal (2) de l'élément tubulaire (1) et le segment distal (3), **caractérisé en ce que** la longueur du segment proximal (2) retourné de l'élément tubulaire (1) correspond au moins à la longueur de l'endoprothèse vasculaire (8) et dans lequel les embranchements (6, 7) sont disposés au moins en partie à l'intérieur de l'endoprothèse vasculaire (8).

2. Implant multi-lumière selon la revendication 1, **caractérisé en ce que** l'élément tubulaire (1) dans le segment distal (3) se ramifie en un embranchement de grande taille (6) avec une lumière principale (4) et en plusieurs embranchements de plus petite taille (7) avec une lumière secondaire (5).

3. Implant multi-lumière selon la revendication 2, **caractérisé en ce que** les lumières secondaires (5) ont un calibre plus petit que la lumière principale (4).

4. Implant multi-lumière selon l'une quelconque des revendications 1 à 3, **caractérisé en ce que** l'endoprothèse vasculaire (8) est disposée sur le côté intérieur du segment proximal (2) de l'élément tubulaire (1).

5. Implant multi-lumière selon l'une quelconque des revendications 1 à 3, **caractérisé en ce que** l'endoprothèse vasculaire (8) est disposée sur le côté extérieur du segment proximal (2) de l'élément tubulaire (1), dans lequel la première partie du segment proximal de l'élément tubulaire (1) est guidée à l'extérieur vers le côté distal au moins en partie tout autour de la partie proximale de l'endoprothèse vasculaire (8).

6. Implant multi-lumière selon l'une quelconque des revendications précédentes, **caractérisé en ce que** le segment proximal (2) de l'élément tubulaire (1) est plus long que l'endoprothèse vasculaire (8), et que la proportion, dépassant distalement (d) de l'endoprothèse vasculaire (8), du segment proximal (2) est retournée vers l'intérieur autour de l'endoprothèse (8).

7. Implant multi-lumière selon l'une quelconque des revendications précédentes, **caractérisé en ce que** des embranchements (6, 7) ou les lumières (4, 5) du segment distal (3) de l'élément tubulaire (1) se rétrécissent (12) vers le côté distal (d).

8. Implant multi-lumière selon l'une quelconque des revendications précédentes, **caractérisé en ce que** les embranchements (6, 7) disposent sur le côté distal de renforts (10, 11) en particulier annulaires.

9. Implant multi-lumière selon l'une quelconque des revendications précédentes, **caractérisé en ce que** l'élément tubulaire (1) est fixé sur l'endoprothèse (8) vasculaire par serrage, collage, couture ou soudure.

10. Implant multi-lumière selon l'une quelconque des revendications précédentes, **caractérisé en ce que** l'élément tubulaire (1) consiste en ePTFE.

11. Implant multi-lumière selon la revendication 10, **caractérisé en ce que** l'élément tubulaire (1) composé de ePTFE peut être obtenu par filage électrostatique.

12. Implant multi-lumière selon l'une quelconque des revendications précédentes, **caractérisé en ce que** l'endoprothèse vasculaire (8) est une endoprothèse auto-expansible.

13. Implant multi-lumière selon l'une quelconque des revendications 1 à 11, **caractérisé en ce que** l'endoprothèse vasculaire (8) est une endoprothèse expansible par ballonnet.

14. Ensemble comprenant un implant selon les revendications précédentes et un cathéter à ballonnet et/ou un cathéter pour son implantation.

15. Ensemble selon la revendication 14, comprenant en outre un nombre correspondant de greffons d'endoprothèse pour le raccordement des embranchements distaux (6, 7) de l'implant aux vaisseaux distaux.
